# EUROPEAN PATENT SPECIFICATION

(11) **EP 3 323 397 B1**
(45) Date of publication and mention of the grant of the patent: **01.04.2020**
(21) Application number: 16824281.6
(22) Date of filing: 30.06.2016
(51) Int. Cl.: A61F 13/49, A61F 13/494

(54) **CROTCH MEMBER FOR DISPOSABLE UNDERGARMENT, AND DISPOSABLE UNDERGARMENT**
SCHRITTTEIL FÜR WEGWERFUNTERWÄSCHE UND WEGWERFUNTERWÄSCHE
ÉLÉMENT D'ENTREJAMBE POUR SOUS-VÊTEMENT JETABLE, ET SOUS-VÊTEMENT JETABLE

(30) Priority: 13.07.2015 JP 2015140098
(43) Date of publication of application: 23.05.2018
(73) Proprietor: Koyo Corporation, Kanagawa 236-0004 (JP)
(72) Inventor: SHIRAI, Atsuko, Yokohama-shi Kanagawa 236-0004 (JP); MATSUMIYA, Munetada, Yokohama-shi Kanagawa 236-0004 (JP)
(74) Representative: Samson & Partner Patentanwälte mbB
(86) International application number: PCT/JP2016/069407
(87) International publication number: WO 2017/010301

(56) References cited:
- EP-A1- 2 862 549
- WO-A1-2014/153485
- JP-A- 2011 078 646
- JP-A- 2013 169 394
- US-A1- 2012 016 334

## Description

### TECHNICAL FIELD

The present invention relates to a crotch member of disposable undergarment such as a pant-type disposable diaper and pad-holder, and a disposable undergarment that has the crotch member.

### BACKGROUND ART

Conventionally known disposable undergarments, such as a pant-type disposable diaper and pad holder, have a crotch member bonded to and bridging between a ventral member and a dorsal member.

For example, Patent Document 1 discloses a pant-type disposable diaper that has a crotch member with a rectangular shape that is bonded to a ventral member with a rectangular shape and a dorsal member with a rectangular shape.

Patent Document 2 discloses a pant-type disposable diaper that has a crotch member with a rectangular shape that is bonded to a ventral member with a rectangular shape having a trapezoidal inward recess on a portion of the lower edge toward the side of the upper edge and a dorsal member with a rectangular shape having a trapezoidal outward protuberance on a portion of the lower edge toward an opposite side of the upper edge. The ventral member and the dorsal member in Patent Document 2 are formed by web dividing in two parts.

### RELATED ART DOCUMENTS

### PATENT DOCUMENTS

Patent Document 1: Japanese Unexamined Patent Application Publication No. 2011-78646.
Patent Document 2: Japanese Patent No. 3916878.

When a shape of a crotch member of disposable undergarment is a rectangular shape or other strip shapes with uniform width, materials will not be wasted by removing trim pieces from original fabric. However, human body has complicated shapes. Each of human body parts such as the groin at the ventral side, the crotch and buttocks at the dorsal side has a different shape and width respectively. Thus, the crotch members with strip shapes having uniform width cannot provide comfortable fitting feel for wearer, and have a problem causing leakage, especially the leakage of excretion.

### SUMMARY OF THE INVENTION

### PROBLEMS TO BE SOLVED BY THE INVENTION

An object of the present invention is to provide a crotch member with a strip shape that achieves excellent fitting for a wearer and prevents leakage of excretion, and to provide a disposable undergarment that has the crotch member.

### MEANS OF SOLVING THE PROBLEMS

To solve the problem described above, a crotch member of a disposable undergarment according to one aspect of the present invention is the crotch member of a disposable undergarment with a strip shape that is bonded to and bridging between a ventral member and a dorsal member, and includes: a ventral bonded portion bonded on the ventral member in an overlapping manner; a dorsal bonded portion bonded on the dorsal member in an overlapping manner; at least one pair of crotch side edge elastic members, that extend and are bonded along to left and right side edges of the crotch member, and are separated from the ventral bonded portion and the dorsal bonded portion; and at least one pair of ventral connection elastic members, that are bonded to the crotch member at a position closer to the center than the positions where the crotch side edge elastic members are bonded to the crotch member, that overlap with the ventral bonded portion at a ventral end portion of the portion where the ventral connection elastic members are bonded to the crotch member, that do not overlap with the dorsal bonded portion at a dorsal end portion of the portion where the ventral connection elastic members are bonded to the crotch member, and that have higher tensile force than the crotch side edge elastic members.

In another aspect of the present invention, in the crotch member, the dorsal end portions of the ventral connection elastic members are disposed at closer to the ventral bonded portions than the dorsal end portions of the crotch side edge elastic members.

In still another aspect of the present invention, in the crotch member, crotch bottom elastic members are bonded to the crotch member at a position closer to the center than a position where the ventral connection elastic members are bonded to the crotch member.

In yet another aspect of the present invention, in the crotch member, an absorbing body is mounted inside of the crotch member. The absorbing body is disposed at a position not overlapping with any of the elastic members. The ventral connection elastic members extend outside of left and right edges of the largest ventral width portion of the absorbing body. The largest dorsal width of the absorbing body is larger than or equal to the largest ventral width of the absorbing body, but narrower than a left-right width of the crotch member.

A disposable undergarment according to one aspect of the present invention is the disposable undergarment that has any of the crotch members.

Further details, features, and advantages of the present invention will be apparent from the description on preferred exemplary embodiments given with reference to the figures.

### BRIEF DESCRIPTION OF THE DRAWINGS

FIG. 1 is a front view of a crotch member of a disposable undergarment according to one embodiment of the present invention in a state where elastic members are stretched.
FIG. 2 is a schematic cross-sectional view of the crotch member illustrated in FIG. 1 taken along line II-II.
FIG. 3 is a diagram illustrating the disposable undergarment that has the crotch member in an open state with the elastic members in a stretched state.
FIG. 4 is a front view of the disposable undergarment that has the crotch member.
FIG. 5 is a back view of the disposable undergarment that has the crotch member.
FIG. 6 is a front view illustrating a state where the disposable undergarment that has the crotch member is worn.
FIG. 7 is a back view illustrating a state where the disposable undergarment that has the crotch member is worn.
FIG. 8 is a diagram illustrating the disposable undergarment that has the crotch member according to another embodiment in an open state with the elastic members in a stretched state.
FIG. 9 is a diagram illustrating the disposable undergarment that has the crotch member according to still another embodiment in an open state with the elastic members in a stretched state.

### MODE FOR CARRYING OUT THE INVENTION

One exemplary embodiment of the present invention is described below.

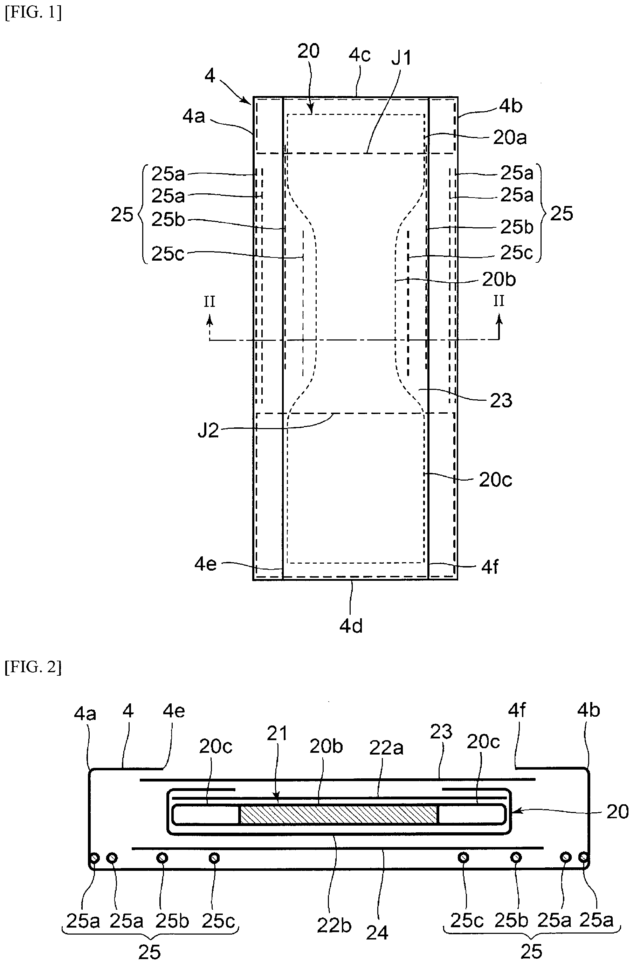

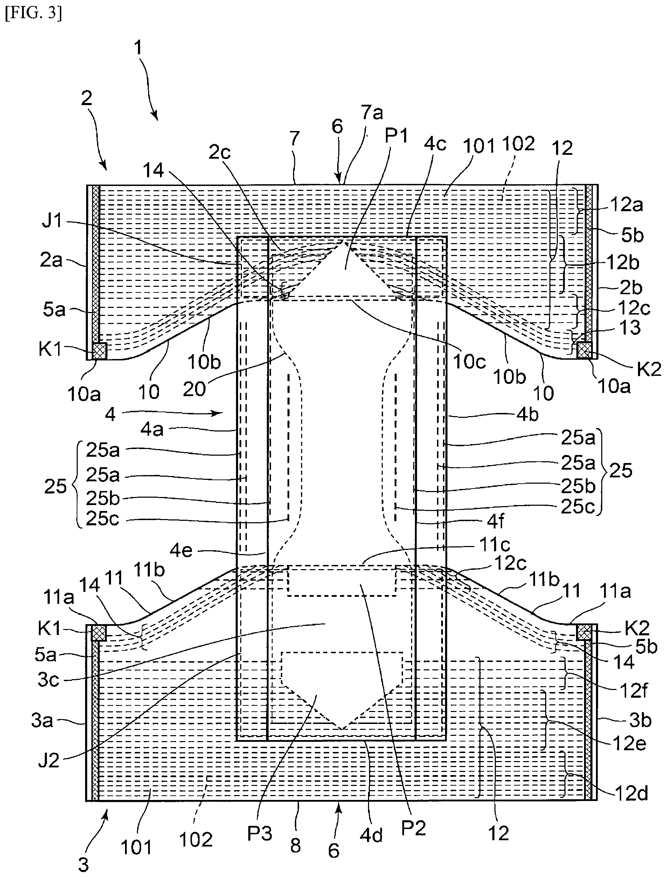

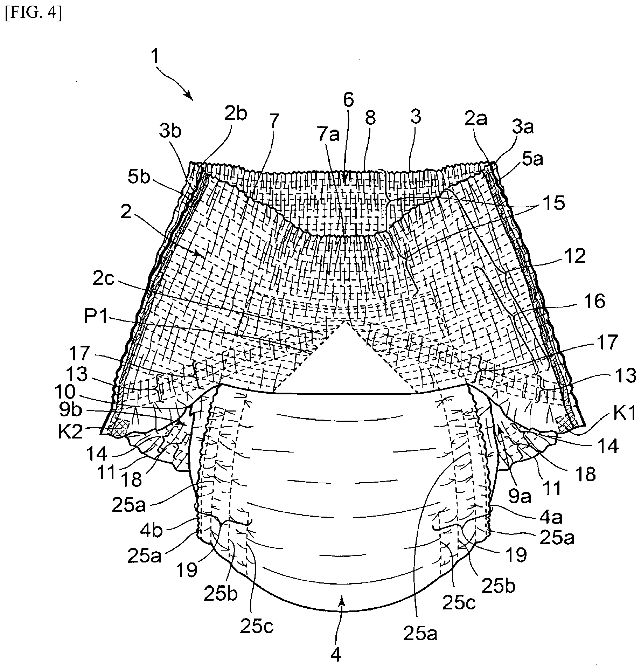

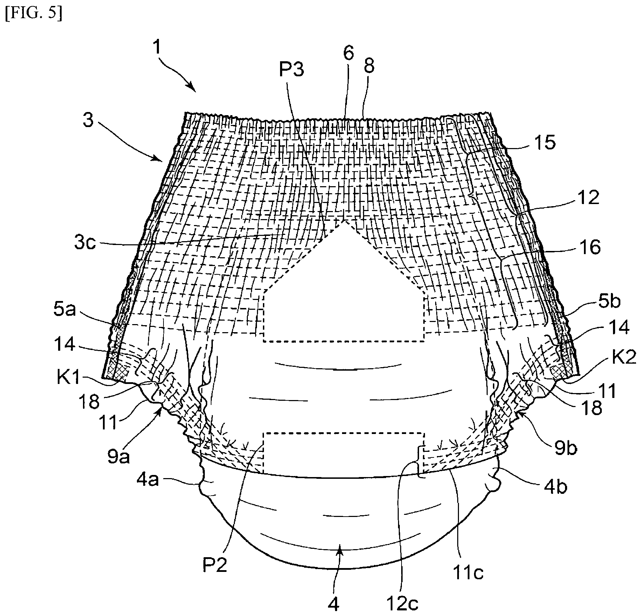

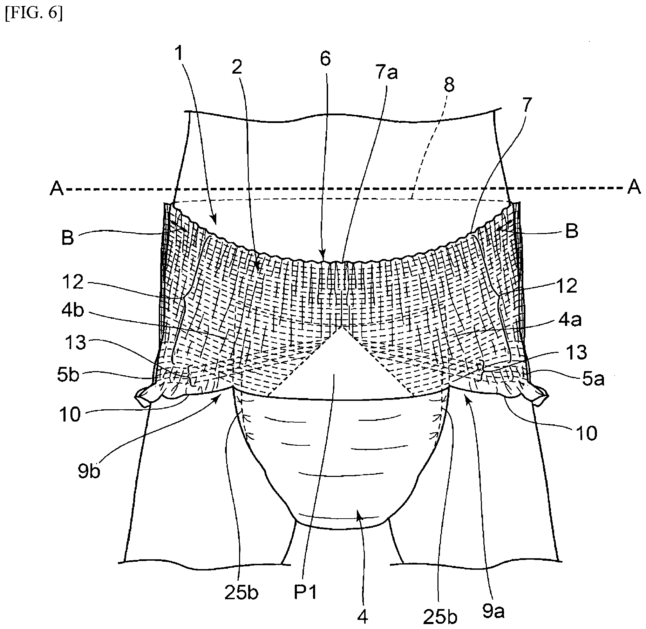

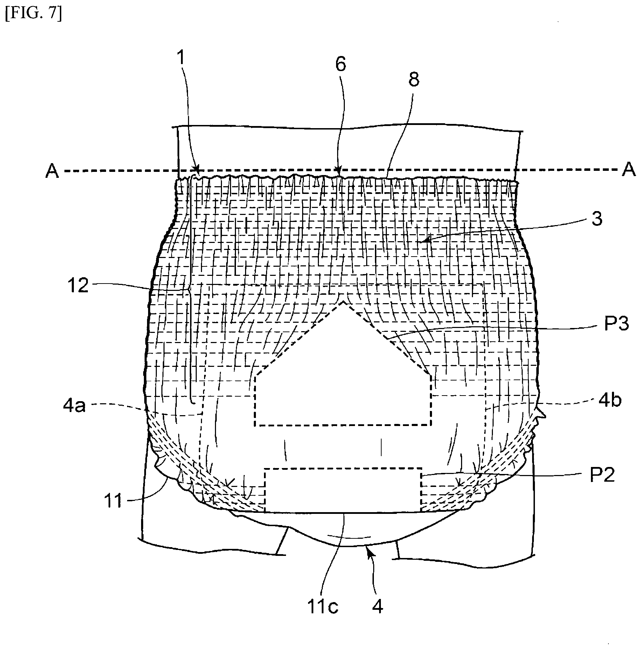

FIG. 1 is a front view of a crotch member 4 according to one embodiment of the present invention in a state where elastic members are stretched. FIG. 2 is a schematic cross-sectional view of the crotch member 4 illustrated in FIG. 1 taken along line II-II. FIG. 3 is a diagram illustrating a disposable undergarment 1 that has the crotch member 4 in an open state with the elastic members in a stretched state. FIG. 4 and FIG. 5 are a front view and a back view of the disposable undergarment 1 that has the crotch member 4. FIG. 6 and FIG. 7 are a front view and a back view illustrating a state where the disposable undergarment 1 that has the crotch member 4 is worn.

As illustrated in FIG. 3 to FIG. 5, the pant-type disposable diaper 1 that has the crotch member 4 according to the present embodiment includes: a ventral member 2 disposed on the wearer's ventral side when the disposable diaper 1 is worn; a dorsal member 3 disposed on the wearer's dorsal side when the disposable diaper 1 is worn; and the crotch member 4 that bridges between and is bonded to center portions 2c and 3c respectively of the ventral member 2 and the dorsal member 3. The ventral member 2 and the dorsal member 3 are bonded to each other at both bonded portions 5a and 5b. The bonded portions 5a and 5b are formed along both end potions 2a and 2b of the ventral member 2 in a waist-around direction and along both end portions 3a and 3b of the dorsal member 3 in the waist-around direction. Positioning bonded portions K1 and K2 are formed in lower portions of the both bonded portions 5a and 5b and a periphery of the lower portions. A waist opening 6 is defined by an upper edge 7 of the ventral member 2 and an upper edge 8 of the dorsal member 3. Left and right leg openings 9a and 9b are each defined by a lower edge 10 of the ventral member 2 or a lower edge 11 of the dorsal member 3 and by a left side edge 4a or a right side edge 4b of the crotch member 4. A hip-surrounding elastic member 12 extends in the waist-around direction in each of the ventral member 2 and the dorsal member 3. A first leg-surrounding elastic member 13 extends in the left and right leg openings 9a and 9b of the ventral member 2. A second leg-surrounding elastic member 14 extends in the left and right leg openings 9a and 9b of the dorsal member 3. Non-elastic areas P1, P2 and P3 are areas where each tensile force of the elastic members 12, 13, and 14 are lost. A crotch elastic member 25 (that is, crotch elastic members 25a, 25b, and 25c) extends at and around the left and right leg openings 9a and 9b of the crotch member 4.

Materials of inner sheets 101 and outer sheets 102 of the disposable diaper 1 are nonwoven fabrics including: chemical fiber such as polyethylene, polypropylene, polyester, nylon, and rayon; natural fiber such as cotton and cellulose; or a combination of these. The material is not limited to the nonwoven fabric and may be a resin film, tissues, an elastic material, a layered body including these, or a sheet member formed by appropriately connecting these.

As illustrated in FIG. 1 and FIG. 2, the crotch member 4 includes: a crotch sheet 4 as the main body of the crotch member; the absorbing body 20 obtained by wrapping an absorbing material 21 with an upper layer tissue 22a and a lower layer tissue 22b; a top sheet 23; a back sheet 24; the crotch elastic member 25 that is appropriately disposed in the crotch sheet 4 while being in the stretched state; and a ventral bonded portion J1 and a dorsal bonded portion J2.

As illustrated in FIG. 3, the crotch sheet 4 as the main body of the crotch member 4 is bonded onto the inner sheets 101 while bridging between the center portion 2c of the ventral member 2 and the center portion 3c of the dorsal member 3. The crotch sheet 4 has the ventral bonded portion J1 and the dorsal bonded portion J2 respectively bonded to the ventral member 2 and the dorsal member 3 with adhesive. The ventral bonded portion J1 is substantially the entire surface overlapping the ventral member 2. The dorsal bonded portion J2 is substantially the entire surface overlapping the dorsal member 3. The material of the crotch sheet 4 is not limited to nonwoven fabrics, and may be the same material with that used for the inner sheets 101 and the outer sheets 102 of the ventral member 2 and the dorsal member 3.

As illustrated in FIG. 1 to FIG. 3, the crotch sheet 4 has a substantially rectangular shape with arranging its longitudinal direction as an extending direction in which the crotch sheet 4 bridges between the ventral member 2 and the dorsal member 3. A ventral side edge 4c of the crotch sheet 4 is positioned between a hip-surrounding elastic member 12a and a hip-surrounding elastic member 12b of the ventral member 2. A dorsal side edge 4d of the crotch sheet 4 is positioned in an upper portion of the area of a hip-surrounding elastic member 12e in the dorsal member 3. Both end portions of the crotch sheet 4 in a width direction are folded toward the absorbing body 20, and the resultant folded lines define left and right side edges 4a and 4b of the crotch member 4. Edges 4e and 4f of the folded pieces of the crotch sheet 4 are positioned outside of left and right edges of the largest width portion of the absorbing body 20 disposed on the crotch sheet 4. However, this should not be construed in a limiting sense, and the edges 4e and 4f of the folded pieces of the crotch sheet 4 may be positioned at inner edges of the absorbing body 20. The width of the crotch member 4, that is, the distance between the left and right side edges 4a and 4b is larger than the largest width of the absorbing body 20. The shape of the crotch sheet 4 is not limited to the substantially rectangular shape, and may be a strip shape with a curved ventral side edge or dorsal side edge. The position and the size of the bonded portion between the crotch member 4 and the ventral member 2 or the dorsal member 3 are not limited to those described above, and may be changed as appropriate in accordance with the size and the shape of the diaper.

As illustrated in FIG. 1 to FIG. 3, the absorbing body 20 is disposed at the center of the crotch sheet 4. The absorbing body 20 has a longitudinal length that is slightly shorter than the longitudinal length of the crotch sheet 4. The length of the absorbing body 20 is preferably a length from a groin upper end to a sacrum upper end of the wearer. The largest width on the ventral side of the absorbing body 20 may preferably be matching with the width of the groin of the wearer. The largest width on the dorsal side of the absorbing body 20 is the same as the largest width on the ventral side. However, this should not be construed in a limiting sense, and the largest width on the dorsal side may be larger than the largest width on the ventral side, and may be narrower than a left-right width of the crotch member 4. The absorbing body 20 has a shape of an hourglass with a narrow portion 20b having the smallest width provided between ventral and dorsal wide portions 20a and 20c having the largest width. The absorbing body 20 is formed as a combination of absorbent paper, cotton pulp, a polymer absorbing material, and the like. And the absorbing body 20 quickly absorbs and holds therein liquid bodies such as urine and blood.

As illustrated in FIG. 2, the absorbing body 20 includes the lower layer tissue 22b, the absorbing material 21, and the upper layer tissue 22a that are stacked in this order from the lower side, and has a structure in which folded pieces of the lower layer tissue 22b are bonded on the upper layer tissue 22a in an overlapping manner. The absorbing material 21 is formed by mixing crushed pulp with the polymer absorbing material. The upper layer tissue 22a is an absorbent paper covering the entire upper surface of the absorbing material 21. The lower layer tissue 22b is an absorbent paper covering the entire lower surface of the absorbing material 21.

As illustrated in FIG. 2, the crotch member 4 includes the crotch sheet 4, the back sheet 24, the absorbing body 20, and the top sheet 23 that are stacked in this order from the lower side, and has a structure in which the folded pieces of the crotch sheet 4 are bonded on the top sheet 23 in an overlapping manner. The top sheet 23 is a sheet member that covers the entire upper surface of the absorbing body 20, and is disposed on an inner side of the disposable diaper 1 to be in contact with the skin of the wearer. For example, the top sheet 23 is made of a liquid permeable material such as nonwoven fabric. Thus, urine, blood, and the like discharged into the diaper 1 transmit through the top sheet 23 to be sent to the absorbing body 20. The back sheet 24 is a sheet member that covers the entire lower surface of the absorbing body 20, and is fixed on the crotch sheet 4. For example, the back sheet 24 is made of a liquid impermeable material such as a polyethylene film. Thus, the liquid body that has transmitted through the top sheet 23 and failed to be absorbed by the absorbing body 20 is prevented from leaking to the crotch sheet 4 or to the outside.

As illustrated in FIG. 1 to FIG. 3, the crotch elastic member 25 includes the crotch side edge elastic members 25a, the ventral connection elastic members 25b, and the crotch bottom elastic members 25c. All the crotch elastic members 25 are disposed while being stretched in the longitudinal direction of the crotch sheet 4. A dotted line portion in each crotch elastic member 25 represents a portion of each crotch elastic member 25 in the stretched state bonded to the crotch sheet 4 with adhesive and the like, and thus is a portion with the tensile force. The crotch elastic member 25 has higher tensile force than the hip-surrounding elastic member 12, the first leg-surrounding elastic member 13, and the second leg-surrounding elastic member 14. The ventral connection elastic member 25b has the highest tensile force in the crotch elastic member 25.

As illustrated in FIG. 1 to FIG. 3, the crotch side edge elastic members 25a are bonded between the crotch sheet 4 and left and right folded pieces of the crotch sheet 4. The portions at where the single crotch side edge elastic member 25a is respectively disposed are as follows: folded lines as the left and right end portions 4a and 4b of the crotch member 4; and portions closer to the center than the aforesaid lines. The crotch side edge elastic member 25a has a portion with the tensile force and is separated from the ventral bonded portion J1 and the dorsal bonded portion J2. The ventral bonded portion J1 is bonded on the ventral member 2 in an overlapping manner. The dorsal bonded portion J2 is bonded on the dorsal member 3 in an overlapping manner. Thus, the crotch side edge elastic member 25a is not connected to the ventral bonded portion J1 and the ventral member 2, and is not connected to the dorsal bonded portion J2 and the dorsal member 3. When the disposable diaper 1 is worn, in the portions of the crotch side edge elastic member 25a, the positions where preferably have the tensile forces are as follows: a ventral end portion is positioned at around the pubic bone of the wearer; and a dorsal end portion is positioned at around the ischial bone.

As illustrated in FIG. 1 to FIG. 3, the ventral connection elastic members 25b are bonded between the crotch sheet 4 and the back sheet 24 with adhesive and the like. The single ventral connection elastic member 25b is disposed at the left and right portions of the crotch side edge elastic member 25a respectively so as to become closer to the center. The ventral connection elastic members 25b are disposed at the left and right outer edge of the largest width portion 20a on the ventral side of the absorbing body 20. The ventral end portion of the portion where the ventral connection elastic member 25b has the tensile force is disposed at a position to be overlapped with the ventral bonded portion J1 of the crotch member 4. Since the crotch member 4 is bonded to the ventral member 2 at the ventral bonded portion J1, the ventral connection elastic member 25b is connected to the ventral member 2. The dorsal end portion of the ventral connection elastic member 25b does not overlap with the dorsal bonded portion J2 of the crotch member 4 and is not connected to the dorsal member 3. The dorsal end portion of the ventral connection elastic member 25b is positioned closer to the ventral bonded portion J1 than the dorsal end portion of the crotch side edge elastic member 25a. When the disposable diaper 1 is worn, in the portions of the ventral connection elastic member 25b, the positions where preferably have the tensile forces are as follows: a ventral end portion is positioned at the groin upper portion of the wearer; and a dorsal end portion is positioned at around the ischial bone.

As illustrated in FIG. 4 to FIG. 6, the tensile force of the ventral connection elastic members 25b connected to the ventral member 2 acts in a direction crossing the hip-surrounding elastic member 12 and the first leg-surrounding elastic member 13 of the ventral member 2. Thus, the ventral member 2 is pulled toward the crotch member 4, whereby the center portion 7a of the upper edge 7 of the ventral member 2 is pulled down.

As illustrated in FIG. 3, FIG. 4 and FIG. 6, when the disposable diaper 1 is worn by the wearer, the pair of ventral connection elastic members 25b and 25b of the crotch member 4 is connected to the ventral member 2, and the ventral member 2 and the crotch member 4 are pulled toward each other. Thus, excellent fitting can be achieved for the wearer. The pair of ventral connection elastic members 25b and 25b is positioned outside of left and right edges of the largest width portion on the ventral side of the absorbing body 20, and is positioned both outside of the groin of the wearer. The crotch side edge elastic members 25a and the ventral connection elastic members 25b are bonded to the left and right side portions of the crotch member 4, whereby gathers are formed. The left and right side portions of the crotch member 4 where the gathers are formed, stand like both side walls due to the tensile force of the ventral connection elastic member 25b connected to the ventral member 2, and thus function as standing cuff. The crotch side edge elastic member 25a is not connected to the ventral member 2 or to the dorsal member 3. Thus, the width of the crotch member 4 can be increased/decreased in accordance with the body shape and the movement of the wearer. Thus, the crotch member 4 has a standing form to cover side and bottom surfaces of the portion from the ventral side to the crotch of the wearer, whereby excellent fitting can be achieved. The standing form of the crotch member 4 is maintained by the tensile force of the crotch side edge elastic member 25a and the ventral connection elastic member 25b. Thus, the excretion can be prevented from leaking through the left and right side edges 4a and 4b of the crotch member 4.

As illustrated in FIG. 3, the portion of the crotch member 4 bonded to the dorsal member 3 has a larger width than the largest width on the dorsal side of the absorbing body 20. In the present embodiment, the absorbing body 20 has the largest width on the dorsal side that is the same as the largest width on the ventral side. However, this should not be construed in a limiting sense, and the largest width on the dorsal side may be larger than the largest width on the dorsal side. The crotch elastic member 25 is not connected to the dorsal member 3. Thus, the portion of the crotch member 4 connected to the dorsal member 3 is not moved by the contraction of the crotch elastic member 25 due to the movement of the wearer. Thus, as illustrated in FIG. 5 and FIG. 7, the disposable diaper 1 can cover the buttocks of the wearer with the large width on the dorsal side of the crotch member 4.

As illustrated in FIG. 1 to FIG. 3, the crotch bottom elastic members 25c are bonded between the crotch sheet 4 and the back sheet 24. The single crotch bottom elastic member 25c is respectively disposed at a portion closer to the center than the ventral connection elastic member 25b and 25b. A portion with the tensile force in the crotch bottom elastic member 25c is preferably disposed in accordance with the shape of the absorbing body disposed on the crotch member 4. When the absorbing body 20 has the shape of an hourglass, the crotch bottom elastic members 25c are disposed in gap areas between the left and right side edges of the narrow portion 20b as the smallest width portion of the absorbing body 20 and the ventral connection elastic members 25b positioned outside of left and right edges of the largest width portion on the ventral side of the absorbing body 20, without overlapping with the absorbing body 20. With the crotch bottom elastic members 25c, gathers are formed in the gap areas, whereby the bottom portion of the crotch member 4 is prevented from loosening and is fit to the wearer, and a static shape of the bottom portion of the crotch member 4 is achieved. The number of crotch bottom elastic members 25c formed on each of the left and right sides is not limited to one and may be more than one. The crotch bottom elastic member 25c may be omitted when the difference between the largest width on the ventral side and the smallest width on the ventral side of the absorbing body 20 is small and thus the gap area is small.

In the present embodiment, two crotch side edge elastic members 25a, one ventral connection elastic member 25b, and one crotch bottom elastic member 25c are formed on each of left and right sides. However, this should not be construed in a limiting sense.

The crotch elastic member 25 is made of a flexible thread-like material such as natural rubber, polyurethane resin, and flexible hot melt, as in the case of the hip-surrounding elastic member 12, the first leg-surrounding elastic member 13, and the second leg-surrounding elastic member 14. The elastic member is not limited to the thread-like form, and an elastic member in a strip form or a sheet form may be used. The entire area where the crotch gathers 19 are formed may be made of a material such as a flexible sheet. When the elastic member in a sheet form is used or when the entire area where the crotch gathers 19 are formed is made of a material such as a flexible sheet, the tensile force acting in an extending direction of each crotch elastic member 25 in a thread form is required.

As described above, in accordance with the present embodiment, the crotch member 4 with a strip shape that achieves excellent fitting for the wearer and prevents leakage of excretion, and the disposable diaper 1 that has the crotch member 4, can be provided.

Next, a crotch member according to another embodiment of the present invention and a disposable undergarment that has the crotch member are described.

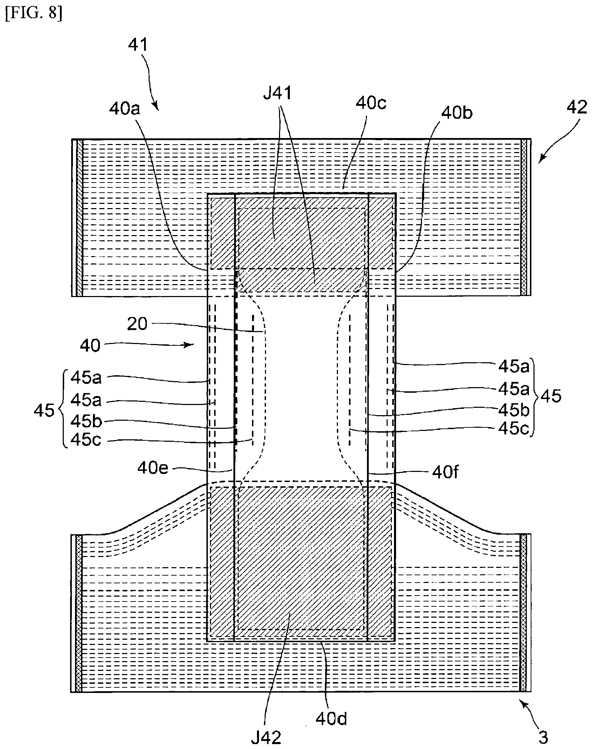

FIG. 8 is a diagram illustrating a disposable undergarment 41 that has a crotch member 40 according to the present embodiment in an open state with the elastic member in a stretched state. Components with configurations and functions that are the same as those in the embodiment as mentioned above of the crotch member 4 and the disposable undergarment 1 are denoted with the same reference numerals, or the redundant description is omitted as appropriate. In the crotch member 4 according to the embodiment as mentioned above, as illustrated in FIG. 3, the ventral member 2 in the stretched state has a rectangular shape with one longitudinal side recessed inward in a substantially trapezoidal form and is used in the disposable undergarment 1. Meanwhile, in the crotch member 40 according to the present embodiment, as illustrated in FIG. 8, the ventral member 42 has a simple rectangular shape and is used in the disposable undergarment 41.

, As illustrated in FIG. 8, in the crotch member 40 according to the present embodiment, the length between the overlapping portion of the ventral member 42 and the overlapping portion of the dorsal member 3 is shorter than the corresponding length in the crotch member 4 of the disposable undergarment 1 as mentioned above. Accordingly, a crotch side edge elastic member 45a and a ventral connection elastic member 45b of the crotch member 40 become shortened. A ventral bonded portion J41 is formed so as to cover almost the whole area where the crotch member 40 overlaps the ventral member 42, but excluding blank areas formed between left and right side edges 40a and 40b of the crotch member 40 and the ventral connection elastic members 45b and 45b. The blank areas are gap areas formed between the crotch side edge elastic member 45a and the ventral bonded portion J41. Also gap areas are formed between the crotch side edge elastic member 45a and the dorsal bonded portion J42.

In the crotch member 40, the pair of ventral connection elastic members 45b and 45b is connected to the ventral member 42, and the ventral member 42 and the crotch member 40 are pulled toward each other. Thus, excellent fitting can be achieved for the wearer. The pair of ventral connection elastic members 45b and 45b is positioned outside of left and right edges of the largest width portion on the ventral side of the absorbing body 20, and is positioned both outside of the groin of the wearer. Along the longitudinal direction of the crotch member 4, the crotch side edge elastic members 45a and the ventral connection elastic members 45b are bonded to the left and right side portions of the crotch member 40, whereby gathers are formed. The left and right side portions of the crotch member 40 where the gathers are formed, stand like both side walls due to the tensile force of the ventral connection elastic member 45b connected to the ventral member 42, and thus function as standing cuff. The crotch side edge elastic member 45a is not connected to the ventral member 42 or to the dorsal member 3. Thus, the width of the crotch member 40 can be increased/decreased in accordance with the body shape and the movement of the wearer. Thus, the crotch member 40 has a standing form to cover side and bottom surfaces of the portion from the ventral side to the crotch of the wearer, whereby excellent fitting can be achieved. The standing form of the crotch member 40 is maintained by the tensile force of the crotch side edge elastic member 45a and the ventral connection elastic member 45b. Thus, the excretion can be prevented from leaking through the left and right side edges 40a and 40b of the crotch member 40.

Next, a crotch member according to still another embodiment of the present invention and a disposable undergarment that has the crotch member are described.

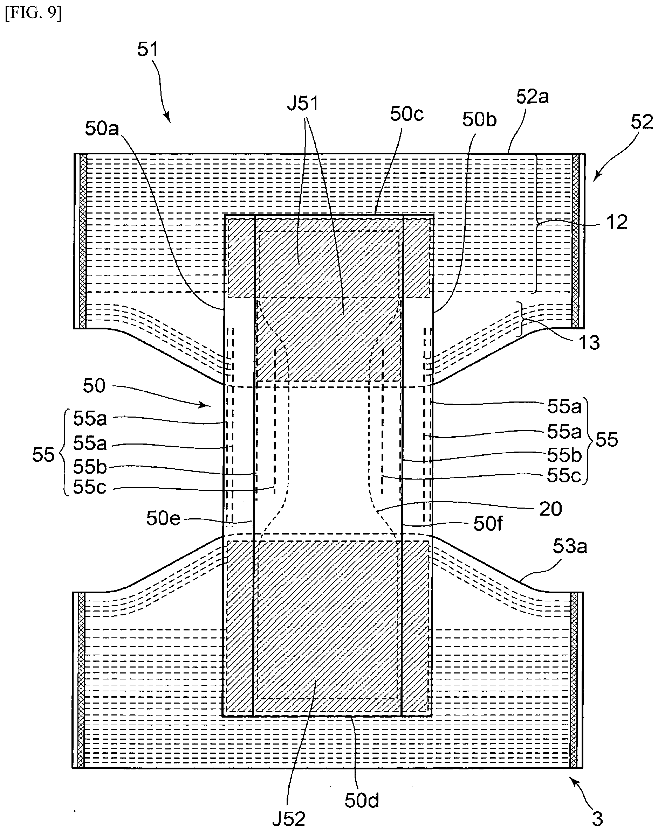

FIG. 9 is a diagram illustrating a disposable undergarment 51 that has a crotch member 50 according to the present embodiment in an open state with the elastic member in a stretched state. Components with configurations and functions that are the same as those in the embodiment as mentioned above of the crotch member 4 and the disposable undergarment 1 are denoted with the same reference numerals, or the redundant description is omitted as appropriate. In the crotch member 50 according to the present embodiment, as illustrated in FIG. 9, a ventral member 52 in the stretched state has a rectangular shape with one longitudinal side protuberance outward in a substantially trapezoidal form and is used in the disposable undergarment 51.

The size of a crotch sheet 50 as the main body of the crotch member 50 according to the present embodiment is the same with the crotch sheet 4 as the main body of the crotch member 4 according to the embodiment as mentioned above. The length between a ventral side edge 50c of the crotch member 50 and an upper edge 52a of the ventral member 52 is the same with the corresponding length in the crotch member 4. Ventral end portions of ventral connection elastic members 55b and 55b are positioned just below the areas where the hip-surrounding elastic member 12 of the ventral member 52 extends to. A ventral bonded portion J51 of the crotch member 50 is formed so as to cover almost the whole large area where the crotch member 50 overlaps the ventral member 52, but excluding blank areas formed between left and right side edges 50a and 50b of the crotch member 50 and the ventral connection elastic members 55b and 55b. The blank areas separate a crotch side edge elastic member 55a from the ventral bonded portion J51.

In the crotch member 50, the pair of ventral connection elastic members 55b and 55b is connected to the ventral member 52, and the ventral member 52 and the crotch member 50 are pulled toward each other. Thus, excellent fitting can be achieved for the wearer. The pair of ventral connection elastic members 55b and 55b is positioned outside of left and right edges of the largest width portion on the ventral side of the absorbing body 20, and is positioned both outside of the groin of the wearer. The crotch side edge elastic members 55a and the ventral connection elastic members 55b are bonded to the left and right side portions of the crotch member 50, whereby gathers are formed. The left and right side portions of the crotch member 50 where the gathers are formed, stand like both side walls due to the tensile force of the ventral connection elastic member 55b connected to the ventral member 52, and thus function as standing cuff. The crotch side edge elastic member 55a is not connected to the ventral member 52 or to the dorsal member 3. Thus, the width of the crotch member 50 can be increased/decreased in accordance with the body shape and the movement of the wearer. Thus, the crotch member 50 has a standing form to cover side and bottom surfaces of the portion from the ventral side to the crotch of the wearer, whereby excellent fitting can be achieved. The standing form of the crotch member 50 is maintained by the tensile force of the crotch side edge elastic member 55a and the ventral connection elastic member 55b. Thus, the excretion can be prevented from leaking through the left and right side edges 50a and 50b of the crotch member 50.

Although not elaborated herein, each of the embodiments and the modifications as mentioned above can be combined as appropriate. The present invention is not limited to any of the embodiments and the modifications as mentioned above. For example, the largest width on the dorsal side of the absorbing body may be larger than the largest width on the ventral side of the absorbing body, and may be within a left-right width of the crotch member. For example, the crotch member according to the present invention may have no absorbing body inside. Further, the disposable undergarment that has the crotch member according to the present invention may be a pad holder that has no absorbing body inside but used with an absorption pad mounted the absorbing body thereon.

### Industrial Applicability

The present invention is suitably used for producing a pant-type disposable diaper and pad holder that has a crotch member of a strip shape that bridges between a ventral member and a dorsal member.

### DESCRIPTION OF THE REFERENCE NUMERAL

1, 41, 51: Disposable undergarment
2, 42, 52: Ventral member
3: Dorsal member
4, 40, 50: Crotch member
20: Absorbing body
25, 45, 55: Crotch elastic member
25a, 45a, 55a: Crotch side edge elastic member
25b, 45b, 55b: Ventral connection elastic member
25c, 45c, 55c: Crotch bottom elastic member
J1, J41, J51: Ventral bonded portion
J2, J42, J52: Dorsal bonded portion

## Claims

1. A crotch member of a disposable undergarment with a strip shape that is bonded to and bridging between a ventral member and a dorsal member comprising:
a ventral bonded portion bonded on the ventral member in an overlapping manner;
a dorsal bonded portion bonded on the dorsal member in an overlapping manner;
at least one pair of crotch side edge elastic members,
wherein the crotch side edge elastic members extend and are bonded along to left and right side edges of the crotch member, and
wherein the crotch side edge elastic members are separated from the ventral bonded portion and the dorsal bonded portion; and
at least one pair of ventral connection elastic members,
wherein the ventral connection elastic members are bonded to the crotch member at a position closer to the center than the positions where the crotch side edge elastic members are bonded to the crotch member,
wherein the ventral connection elastic members overlap with the ventral bonded portion at a ventral end portion of the portion where the ventral connection elastic members are bonded to the crotch member,
wherein the ventral connection elastic members do not overlap with the dorsal bonded portion at a dorsal end portion of the portion where the ventral connection elastic members are bonded to the crotch member, and
wherein the ventral connection elastic members have higher tensile force than the crotch side edge elastic members.

2. The crotch member of the disposable undergarment according to claim 1,
wherein the dorsal end portions of the ventral connection elastic members are disposed at closer to the ventral bonded portions than dorsal end portions of the crotch side edge elastic members.

3. The crotch member of the disposable undergarment according to claim 1,
wherein crotch bottom elastic members are bonded to the crotch member at a position closer to the center than a position where the ventral connection elastic members are bonded to the crotch member.

4. The crotch member of the disposable undergarment according to any of claims 1 to 3,
wherein an absorbing body is mounted inside of the crotch member,
wherein the absorbing body is disposed at a position not overlapping with any of the elastic members,
wherein the ventral connection elastic members extend outside of left and right edges of the largest ventral width portion of the absorbing body,
wherein the largest dorsal width of the absorbing body is larger than or equal to the largest ventral width of the absorbing body, but narrower than a left-right width of the crotch member.

5. The disposable undergarment that has the crotch member according to any of claims 1 to 4.

## Patentansprüche

1. Schrittelement einer Einwegunterwäsche mit einer Streifenform, das mit einem ventralen Element und einem dorsalen Element verbunden ist und eine Brücke zwischen diesen bildet, umfassend:
einen ventralen gebundenen Abschnitt, der in überlappender Weise mit dem ventralen Element verbunden ist;
einen dorsalen gebundenen Abschnitt, der in überlappender Weise mit dem dorsalen Element verbunden ist;
mindestens ein Paar elastische Elemente an der Schrittseitenkante,
wobei sich die elastischen Elemente der Schrittseitenkante entlang der linken und rechten Seitenkanten des Schrittelements erstrecken und entlang dieser verbunden sind, und
wobei die elastischen Elemente der Schrittseitenkante vom ventralen gebundenen Abschnitt und dem dorsalen gebundenen Abschnitt getrennt sind; und
mindestens ein Paar elastische Elemente der ventralen Verbindung,
wobei die elastischen Elemente der ventralen Verbindung an das Schrittelement an einer Position gebunden sind, die näher an der Mitte liegt als die Positionen, an denen die elastischen Elemente der Schrittseitenkante mit dem Schrittelement verbunden sind,
wobei sich die elastischen Elemente der ventralen Verbindung mit dem ventralen gebundenen Abschnitt an einem ventralen Endabschnitt des Abschnitts überlappen, an dem die elastischen Elemente der ventralen Verbindung mit dem Schrittelement verbunden sind,
wobei die elastischen Elemente der ventralen Verbindung nicht mit dem dorsalen gebundenen Abschnitt an einem dorsalen Endabschnitt des Abschnitts überlappen, an dem die elastischen Elemente der ventralen Verbindung mit dem Schrittelement verbunden sind, und
wobei die elastischen Elemente der ventralen Verbindung eine höhere Zugkraft aufweisen als die elastischen Elemente der Schrittseitenkante.

2. Schrittelement der Einwegunterwäsche nach Anspruch 1,
wobei die dorsalen Endabschnitte der elastischen Elemente der ventralen Verbindung näher an den ventralen gebundenen Abschnitten angeordnet sind als die dorsalen Endabschnitte der elastischen Elemente der Schrittseitenkante.

3. Schrittelement der Einwegunterwäsche nach Anspruch 1,
wobei die elastischen Elemente des Schrittbodens mit dem Schrittelement in einer Position verbunden sind, die näher an der Mitte liegt als eine Position, in der die elastischen Elemente der ventralen Verbindung mit dem Schrittelement verbunden sind.

4. Schrittelement der Einwegunterwäsche nach einem der Ansprüche 1 bis 3,
wobei ein absorbierender Körper innerhalb des Schrittelements montiert ist,
wobei der absorbierende Körper an einer Position angeordnet ist, die sich nicht mit einem der elastischen Elemente überlappt,
wobei sich die elastischen Elemente der ventralen Verbindung außerhalb der linken und rechten Kanten des größten ventralen Breitenabschnitts des absorbierenden Körpers erstrecken,
wobei die größte dorsale Breite des absorbierenden Körpers größer oder gleich der größten ventralen Breite des absorbierenden Körpers ist, aber schmaler als eine links-rechts Breite des Schrittelements.

5. Einwegunterwäsche, die das Schrittelement nach einem der Ansprüche 1 bis 4 aufweist.

## Revendications

1. Élément d'entrejambe d'un sous-vêtement jetable ayant une forme de bande qui est lié à un élément ventral et à un élément dorsal et fait la liaison entre ceux-ci comprenant :
une partie liée ventrale liée sur l'élément ventral en chevauchement de l'élément ventral;
une partie liée dorsale liée sur l'élément dorsal en chevauchement de l'élément dorsal ;
au moins une paire d'éléments élastiques de bord latéral d'entrejambe,
dans lequel les éléments élastiques de bord latéral d'entrejambe s'étendent et sont liés le long de bords latéraux gauche et droit de l'élément d'entrejambe, et
dans lequel les éléments élastiques de bord latéral d'entrejambe sont séparés de la partie liée ventrale et de la partie liée dorsale ; et
au moins une paire d'éléments élastiques de liaison ventraux,
dans lequel les éléments élastiques de liaison ventraux sont liés à l'élément d'entrejambe dans une position plus proche du centre que les positions où les éléments élastiques de bord latéral d'entrejambe sont liés à l'élément d'entrejambe,
dans lequel les éléments élastiques de liaison ventraux chevauchent la partie liée ventrale au niveau d'une partie d'extrémité ventrale de la partie où les éléments élastiques de liaison ventraux sont liés à l'élément d'entrejambe,
dans lequel les éléments élastiques de liaison ventraux ne chevauchent pas la partie liée dorsale au niveau d'une partie d'extrémité dorsale de la partie où les éléments élastiques de liaison ventraux sont liés à l'élément d'entrejambe, et
dans lequel les éléments élastiques de liaison ventraux présentent une force de traction plus élevée que les éléments élastiques de bord latéral d'entrejambe.

2. Élément d'entrejambe du sous-vêtement jetable selon la revendication 1,
dans lequel les parties d'extrémité dorsales des éléments élastiques de liaison ventrales sont disposées plus près des parties liées ventrales que les parties d'extrémité dorsales des éléments élastiques de bord latéral d'entrejambe.

3. Élément d'entrejambe du sous-vêtement jetable selon la revendication 1,
dans lequel des éléments élastiques de fond d'entrejambe sont liés à l'élément d'entrejambe dans une position plus proche du centre qu'une position où les éléments élastiques de liaison ventraux sont liés à l'élément d'entrejambe.

4. Élément d'entrejambe du sous-vêtement jetable selon l'une quelconque des revendications 1 à 3,
dans lequel un corps absorbant est monté à l'intérieur de l'élément d'entrejambe,
dans lequel le corps absorbant est disposé dans une position ne chevauchant aucun des éléments élastiques,
dans lequel les éléments élastiques de liaison ventraux s'étendent à l'extérieur de bords gauche et droit de la partie de largeur ventrale la plus grande du corps absorbant,
dans lequel la largeur dorsale la plus grande du corps absorbant est plus grande que la largeur ventrale la plus grande du corps absorbant ou égale à celle-ci, mais plus étroite qu'une largeur gauche-droite de l'élément d'entrejambe.

5. Sous-vêtement jetable qui comporte l'élément d'entrejambe selon l'une quelconque des revendications 1 à 4.
